# EUROPÄISCHE PATENTSCHRIFT

(11) **EP 1 040 857 B1**
(45) Veröffentlichungstag und Bekanntmachung des Hinweises auf die Patenterteilung: **02.06.2004**
(21) Anmeldenummer: 00106086.2
(22) Anmeldetag: 30.03.2000
(51) Int. Cl.: B01D 3/14, C07C 29/80

(54) **Verfahren zur kontinuierlich betriebenen destillativen Abtrennung eines höherschmelzenden Stoffes**
Method for separating a high melting substance by a continuous distillation process
Procédé de séparation par distillation continu d'une substance fondant à haute température

(30) Priorität: 01.04.1999 DE 19914966
(43) Veröffentlichungstag der Anmeldung: 04.10.2000
(73) Patentinhaber: BASF AKTIENGESELLSCHAFT, 67056 Ludwigshafen (DE)
(72) Erfinder: Tragut, Christian, Dr., 2040 Antwerpen (BE); Kaibel, Gerd, Dr., 68623 Lampertheim (DE)
(74) Vertreter: Isenbruck, Günter, Dr.

(56) Entgegenhaltungen:
- EP-A- 0 780 147
- KAIBEL G ET AL: "GESTALTUNG DESTILLATIVER TRENNUNGEN UNTER EINBEZIEHUNG THERMODYNAMISCHER GESICHTSPUNKTE" CHEMIE. INGENIEUR. TECHNIK,DE,VERLAG CHEMIE GMBH. WEINHEIM, Bd. 61, Nr. 1, 1989, Seiten 16-18,21-25, XP000025551 ISSN: 0009-286X

## Beschreibung

Die Erfindung betrifft ein Verfahren zur kontinuierlich betriebenen destillativen Abtrennung eines höherschmelzenden Stoffes aus einem den höherschmelzenden Stoff und niedrig schmelzenden Leichtsieder enthaltenden Ausgangsgemisch nach Anspruch 1.

In der Regel sind in einem solchen Ausgangsgemisch neben dem Leichtsieder und dem höherschmelzenden Stoff auch noch weitere Komponenten enthalten. Der höherschmelzende Stoff soll meist als mittelsiedendes Wertprodukt aus dem Ausgangsgemisch isoliert werden. Bei der üblichen destillativen Reinigung von mittelsiedenden Wertprodukten von leicht- und schwersiedenden Verunreinigungen sind verschiedene Verfahrensvarianten gebräuchlich. Im einfachsten Fall wird das den höherschmelzenden Stoff aufweisende Zulaufgemisch in zwei Fraktionen, in eine leichtsiedende Kopffraktion und in eine schwersiedende Sumpffraktion, zerlegt. Bei der Auftrennung von Zulaufgemischen in mehr als zwei Fraktionen - zum Beispiel in Leichtsieder, Mittelsieder und Hochsieder - müssen nach dieser Verfahrensvariante mehrere Destillationskolonnen eingesetzt werden. Um den apparativen Aufwand zu begrenzen, setzt man bei der Auftrennung von Vielstoffgemischen, die aus mehr als zwei Komponenten bestehen, auch Kolonnen ein, die sich für die Seitenentnahme flüssiger oder gasförmiger Medien eigenen. Die Anwendungsmöglichkeit von Destillationskolonnen mit Seitenabzügen ist jedoch dadurch stark eingeschränkt, daß die an den Seitenabzugsstellen entnommenen Produkte normalerweise nicht völlig rein sind. Bei Seitenentnahmen im Verstärkungsteil einer Destillationsvorrichtung, die üblicherweise in flüssiger Form erfolgen, enthalten die Seitenprodukte noch Anteile an leichtsiedenden Komponenten, die im Normalfall über Kopf abgetrennt werden. Entsprechendes gilt für Seitenentnahmen im Abtriebsteil, die meist dampfförmig erfolgen und bei denen die entnommenen Seitenprodukte noch Anteile des Hochsieders aufweisen. Bei der Verwendung solcher konventionellen Seitenabzugskolonnen werden praktisch immer verunreinigte Seitenprodukte erhalten - die Verwendung von Seitenabzugskolonnen ist daher zur Gewinnung von Reinstoffen ungeeignet. Insbesondere zur Isolierung von mittelsiedenden Reinstoffen aus Vielstoffgemischen müssen somit in der Regel Kolonnenanordnungen, die aus mindestens zwei separaten Kolonnen bestehen, eingesetzt werden.

Eine vorteilhafte Alternative sind sogenannte Trennwandkolonnen oder thermisch gekoppelte Destillationskolonnen. Der Einsatz von Trennwandkolonnen ermöglicht es, Seitenprodukte, also mittelsiedende Komponenten, ebenfalls in reiner Form aus Vielstoffgemischen zu isolieren. Bei Trennwandkolonnen ist im mittleren Bereich eine Trennwand angebracht. Diese erstreckt sich oberhalb und unterhalb der Zulaufstelle. Auf der anderen Seite, die sich gegenüber der Zulaufstelle befindet, ist eine Seitenabzugstelle angeordnet. Zwischen Seitenabzugstelle und Zulaufstelle befindet sich die Trennwand. In dem Kolonnenbereich, der durch die Trennwand geteilt wird, ist eine Quervermischung von Flüssigkeits- und Brüdenströmen nicht möglich. Dadurch verringert sich bei der Auftrennung von Vielstoffgemischen die Zahl der insgesamt benötigten Destillationskolonnen. Dieser Kolonnentyp ist im Prinzip eine apparative Vereinfachung von thermisch gekoppelten Destillationskolonnen, wobei jedoch letztere höhere Investitionskosten aufweisen. Trennwandkolonnen und thermisch gekoppelte Kolonnen bieten gegenüber der Anordnung von konventionellen Destillationskolonnen sowohl hinsichtlich des Energiebedarfs als auch der Investitionskosten Vorteile und werden daher bevorzugt industriell eingesetzt. Informationen über Trennwandkolonnen bzw. thermisch gekoppelten Destillationskolonnen sind in der EP-A-0 122 367, in der EP-B-0 126 288 und in der EP-B-0 133 510 aufgeführt.
Die destillative Auftrennung eines Vielstoffgemischs, das eine mittelsiedende Komponente enthält, die einen Schmelzpunkt aufweist, der oberhalb der Umgebungstemperatur liegt, erfordert einen erhöhten technischen Aufwand. Es ist in einem solchen Fall nicht in einfacher Weise möglich, die Kondensationswärme am Kopf einer Kolonne über einen Kühler oder über einen mit Kühlwasser oder Rückkühlwasser beaufschlagten Kondensator abzuführen. In einem solchen Fall würde sich der Wärmetauscher - also ein Luftkühler oder ein mit Kühlwasser oder Rückkühlwasser betriebene Kühler - rasch mit Kristallisat belegen, da die Komponente mit dem hohen Schmelzpunkt sich abscheiden würde. Eine Kristallschicht auf einem Kondensator ist nachteilig, da die Wärmeübertragung vermindert wird und der Kondensator dadurch seine Funktion nicht mehr wie gewünscht wahrnehmen kann.

Als Abhilfemöglichkeit kann man eine Anordnung von mehreren Kondensatoren benutzen, die taktweise im Kühlbetrieb arbeiten und wieder abgeschmolzen werden. Diese Arbeitsweise ist aufwendig und erfordert einen hohen Automatisierungsaufwand.

Technisch verbreitet ist die Nutzung eines Sekundärmediums. Als Sekundärmedium kommt beispielsweise thermostatisiertes Öl oder Heißwasser in Frage. Die Temperatur des Sekundärmediums ist so eingestellt, daß eine Kristallbildung auf den Wärmetauscherflächen verhindert wird. In der Regel steht ein solches Sekundärmedium im Kontakt mit einem weiteren Kühlmedium, auf das die Wärme übertragen werden kann. Man benötigt sozusagen zwei Kühlkreisläufe. Dies bedingt erhöhte Investitionskosten.

Ein weiterer Nachteil ergibt sich aus der Verstopfung durch die höherschmelzende Komponente: Letztere kann sich, falls sie am Kondensator nicht abgeschieden wird, in nachgeschalteten Abgasleitungen ablagern. Dies verursacht unerwünschte Druckverluste.

Die Aufgabe der vorliegenden Erfindung ist, ein Verfahren hervorzubringen, mit dem ein höherschmelzender Stoff - also eine höherschmelzende Komponente - aus einem Ausgangsgemisch, das mehrere Komponenten enthält, gewonnen werden kann. Dabei soll am entsprechenden Kondensator, der bei der Destillation verwendet wird, kein Feststoff abgeschieden werden. Außerdem soll der höherschmelzende Stoff aus dem entsprechenden Ausgangsgemisch mit Hilfe eines effektiven und energetisch vorteilhaften Destillationsverfahrens gewonnen werden.

Die Lösung dieser Aufgabe geht aus von einem Verfahren zur kontinuierlich betriebenen destillativen Abtrennung eines höherschmelzenden Stoffes aus einem den höherschmelzenden Stoff und *einen* niedrig schmelzenden Leichtsieder enthaltenden Ausgangsgemisch, wobei der *niedrig schmelzende* Leichtsieder aus *einer* oder mehreren Komponenten mit einem niedrigeren Siedepunkt als der höherschmelzende Stoff besteht, in einer Destillationsapparatur, die entweder als Trennwandkolonne oder als System thermisch gekoppelter Destillationskolonnen ausgebildet ist. Gelöst wird diese Aufgabe dadurch, daß die Destillationsapparatur mit einem im Kopf, oberhalb der Entnahmestelle des höherschmelzenden Stoffes befindlichen Kondensator ausgestattet ist, dessen Teilbereich der Oberfläche, der mit dem Innenraum der Destillationsapparatur Kontakt hat, eine niedrigere Temperatur aufweist als der Schmelzpunkt des höherschmelzenden Stoffes und der Leichtsieder im Kopf so stark *konzentriert ist*, daß keine Abscheidung des höherschmelzenden Stoffes am Kondensator erfolgt.

"Keine Abscheidung" des höherschmelzenden Stoffes soll in diesem Zusammenhang bedeuten, daß weniger als 1 Gew.-%, bevorzugt weniger als 0,001 Gew.-% des eingesetzten höherschmelzenden Stoffes an dem Kondensator in fester Form abgeschieden wird. Als höherschmelzender Stoff, soll ein Stoff verstanden werden, der einen höheren Schmelzpunkt aufweist als die Temperatur, die im Durchschnitt an der Oberfläche des verwendeten Kondensators vorliegt.

Umgekehrt weist ein niedrigschmelzender Stoff einen Schmelzpunkt auf, der unterhalb der mittleren Oberflächentemperatur des Kondensators liegt. Ein niedrigschmelzender Stoff kann sich somit nicht an einem entsprechenden Kondensator als Feststoff abscheiden. Niedrigschmelzend und höherschmelzend sind somit relative Eigenschaften. Je nach Destillationsverfahren, das heißt je nach der Oberflächentemperatur des eingesetzten Kondensators, kann der gleiche Stoff höherschmelzend und gleichzeitig auch niedrigschmelzend sein.

Unter Verfahrensbedingungen sollen die Konzentrationen der in der Destillationsapparatur enthaltenen Stoffe - bzw. die Hinzugabe und Entnahme der Stoffe, die Oberflächentemperatur des Kondensators, die an den verschiedenen Stellen der Destillationsapparatur vorliegenden Drücke und Temperaturen und die Art und Anzahl der Trennstufen der Destillationsapparatur, verstanden werden.

Bevorzugt enthält die Destillationsapparatur nur einen Kondensator. Dieser befindet sich im Kopf der Kolonne, oberhalb der Entnahmestelle des höherschmelzenden Stoffes. Die Entnahmestelle des höherschmelzenden Stoffes liegt bei einer Trennwandkolonne in der Regel unterhalb des oberen Endes der Trennwand. Bei thermisch gekoppelten Destillationskolonnen befindet sich die Entnahmestelle des höherschmelzenden Stoffes nicht in der gleichen Kolonne, in der das Ausgangsgemisch aufgegeben wird, sondern in einer zweiten Kolonne. Letztere ist durch Verbindungsleitungen mit der Kolonne verbunden, in der das Ausgangsgemisch aufgegeben wird. Bei thermisch gekoppelten Destillationskolonnen ist die die Entnahmestelle des höherschmelzenden Stoffes auch unterhalb des Kondensators angeordnet. Das bedeutet in diesem Fall, daß der Kondensator oberhalb der Fügungsstelle angeordnet ist, durch die die oberste Verbindungsleitung zwischen den beiden gekoppelten Kolonnen mit der den Kondensator aufweisenden Kolonne, verbunden ist.

In einer bevorzugten Ausführungsform enthält das Ausgangsgemisch neben dem höherschmelzenden Stoff und dem Leichtsieder zusätzlich noch einen aus einer oder mehreren Komponenten bestehenden Schwersieder, wobei alle Komponenten des Schwersieders einen höheren Siedepunkt als der höherschmelzende Stoff aufweisen. In einem solchen Fall ist der höherschmelzende Stoff ein Mittelsieder, der neben Leichtsieder und Schwersieder im Ausgangsgemisch vorliegt. Prinzipiell können neben Leichtsieder, Schwersieder und dem höherschmelzenden Stoff auch noch andere Bestandteile in dem Ausgangsgemisch enthalten sein, die meist entweder nicht destilliert werden oder einen ähnlichen Siedepunkt wie der höherschmelzende Stoff aufweisen. Der höherschmelzende Stoff ist in der Regel das Wertprodukt der destillativen Trennung und wird, falls eine Trennwandkolonne eingesetzt wird, als Seitenabzugsprodukt gewonnen.

In der Regel liegt nur eine Teilmenge des Leichtsieders im Ausgangsgemisch als Fremdstoff, also als zusätzlich, allein zum Zweck der destillativen Trennung zugesetzte Komponente, vor. Das bedeutet zum Beispiel, daß bei einem Gehalt an leichtsiedenden Verunreinigungen in dem roh vorliegenden höherschmelzenden Stoff der Zusatz weiterer Leichtsieder - vorstehend als Fremdstoffe bezeichnet - reduziert werden kann. Sind die als Leichtsieder fungierenden Verunreinigungen in ausreichender Konzentration vorhanden, braucht kein weiterer Leichtsieder (Fremdstoff) zum Ausgangsgemisch hinzugefügt werden. Der Verzicht auf Fremdstoffe als verfahrenstechnische Zusatzstoffe ist von großem Vorteil, da letztere nachträglich wieder abgetrennt werden müssen. Der damit verbundene zusätzlicher Trennaufwand wirkt sich negativ auf die Wirtschaftlichkeit des entsprechenden Verfahrens aus.

In dem erfindungsgemäßen Verfahren eignen sich als höherschmelzende Stoffe beispielsweise Isomere des Dimethylhexandiols. Als Leichtsieder können niedrig siedende Alkohole und/oder niedrig siedende aromatische Kohlenwasserstoffe und/oder niedrig siedende Ether eingesetzt werden.

In der Regel ist die Destillationsapparatur entweder als Packungskolonne mit Füllkörpern oder geordneten Packungen oder als Bodenkolonne ausgebildet. Häufig empfiehlt es sich, insbesondere wenn thermische Empfindlichkeit und niedrige Siedepunkte der zu trennenden Substanzen vorliegen, die Destillation im Vakuum durchzuführen. In einem solchen Fall (beispielsweise bei der Destillation von Dimethylhexandiol, die bevorzugt bei einem Druck von 50 bis 300 mbar durchgeführt wird) sind druckverlustarme Packungskolonnen vorteilhaft. Dabei sind geordnete Gewebepackungen mit einer spezifischen Oberfläche von 200 bis 800 m²/m³, bevorzugt 400 bis 600 m²/m³, von Vorteil.

Der Leichtsieder kann als Lösungsmittel für den höherschmelzenden Stoff fungieren. An dem Kondensator abgeschiedene Kristalle des höherschmelzenden Stoffes würden dann von dem Leichtsieder fortwährend gelöst und dadurch von dem Kondensor entfernt. Eine weitere Möglichkeit ist, daß der Leichtsieder den Schmelzpunkt des höherschmelzenden Stoffes reduziert, zum Beispiel durch die Bildung eines eutektischen Gemischs. Ist die Konzentration des Leichtsieders im Bereich des Kondensators zu gering, so werden vorstehende Effekte nicht in genügendem Maße wirksam, so daß sich der höherschmelzende Stoff am Kondensator abscheidet.

In einer bevorzugten Ausführungsform wird die notwendige Anreicherung des Leichtsieders im Kopf durch
a) eine ausreichend groß bemessene Trennstufenzahl im oberen Bereich der Destillationsapparatur und/oder
b) durch eine hinreichend starke Beheizung der Destillationsapparatur und/oder
c) durch eine kontrollierte Entnahme des Leichtsieders am Kopf erreicht.

Mit dem oberen Bereich der Destillationsapparatur ist bei einer Trennwandkolonne der Bereich gemeint, der oberhalb der Trennwand liegt - bei einem System thermisch gekoppelter Destillationskolonnen der Bereich, der sich oberhalb der Fügungsstelle befindet, durch die die oberste Verbindungsleitung zwischen den beiden gekoppelten Kolonnen mit der den Kondensator aufweisenden Kolonne, verbunden ist.

In der Regel wird die Anreicherung des Leichtsieders im Kopf durch eine Temperaturregelung im oberen Bereich der Destillationsapparatur gesteuert. Zur Gewährleistung von genügenden Mengen Leichtsieder im Kopf der Destillationsapparatur bzw. zur Vermeidung von Abscheidung des höherschmelzenden Stoffes an dem Kondensator erweisen sich ein oder mehrere Punkte des folgenden Regelungskonzepts als besonders günstig:
- Eine Temperaturregelung im oberen Bereich der Destillationsapparatur ist eingerichtet, die als Stellgröße die Ablaufmenge, das Rücklaufverhältnis oder bevorzugt die Rücklaufmenge nutzt - diese Temperaturregelung kann so ergänzt werden, daß bei einer Temperaturüberschreitung und gleichzeitigem Stelleingriff, der einem unendlich hohen Rücklaufverhältnis entspricht, zusätzlich Leichtsieder auf den Kopf der Kolonne aufgegeben wird.
- Eine Temperaturregelung im Bereich des Sumpfes ist eingerichtet, die als Stellgröße die Sumpfentnahmemenge nutzt.
- Im Falle der Verwendung einer Trennwandkolonne als Destillationsapparatur wird der nach oben aufsteigende Dampfstrom auf beide Seiten der Trennwand in einem Verhältnis von 0,8 : 1,2 (v/v) bis 1,2 : 0,8 (v/v) verteilt.

Zur Entnahme des höherschmelzenden Stoffs an der Seitenabzugsstelle eignen sich sowohl innenliegende als auch außerhalb der Kolonne angeordnete Auffangräume. Diese übernehmen in der Regel die Funktion einer Pumpenvorlage oder sorgen jeweils für eine ausreichend hohe statische Flüssigkeitshöhe. Letztere gewährleistet eine durch Stellorgane, beispielsweise Ventile, geregelte Flüssigkeitsweiterleitung.

In der anliegenden Zeichnung sind dargestellt:

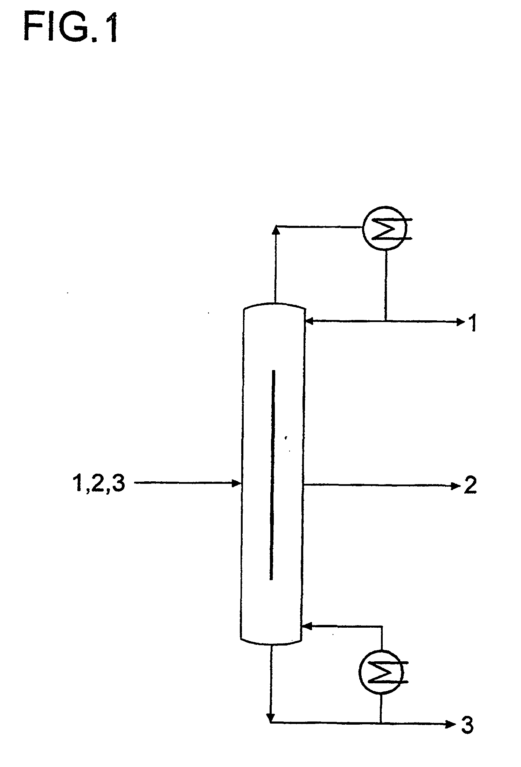

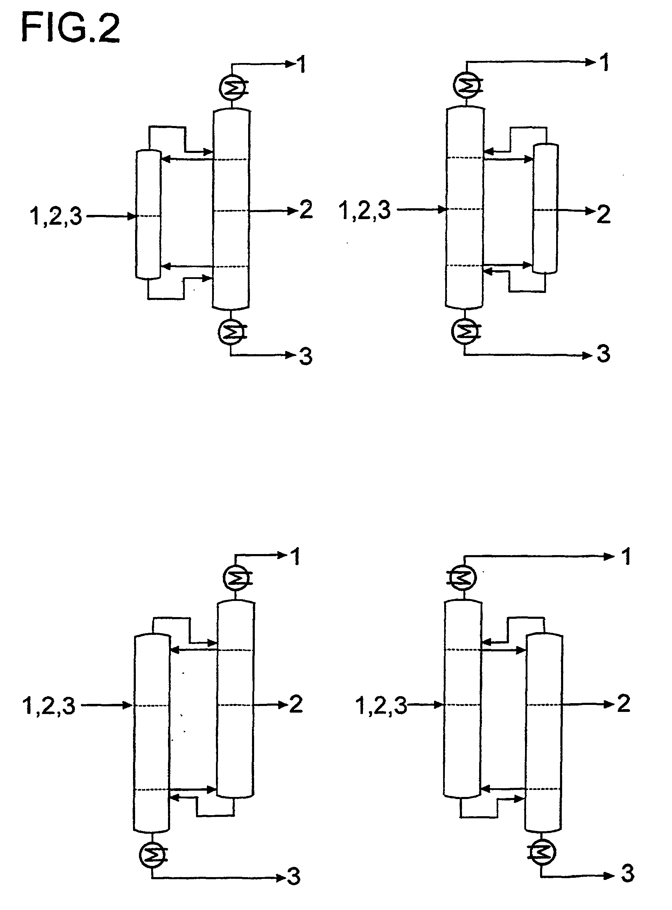

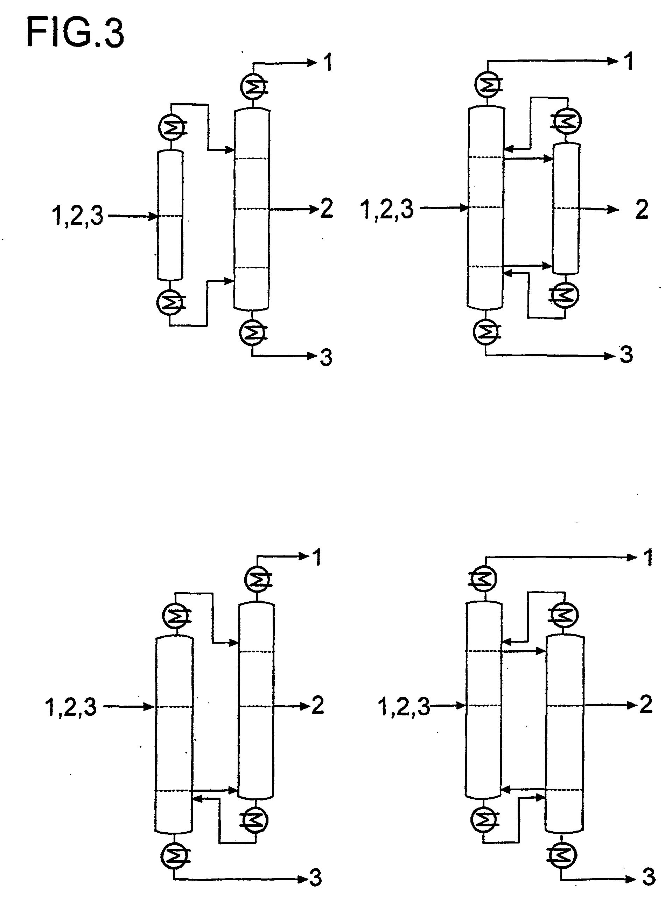

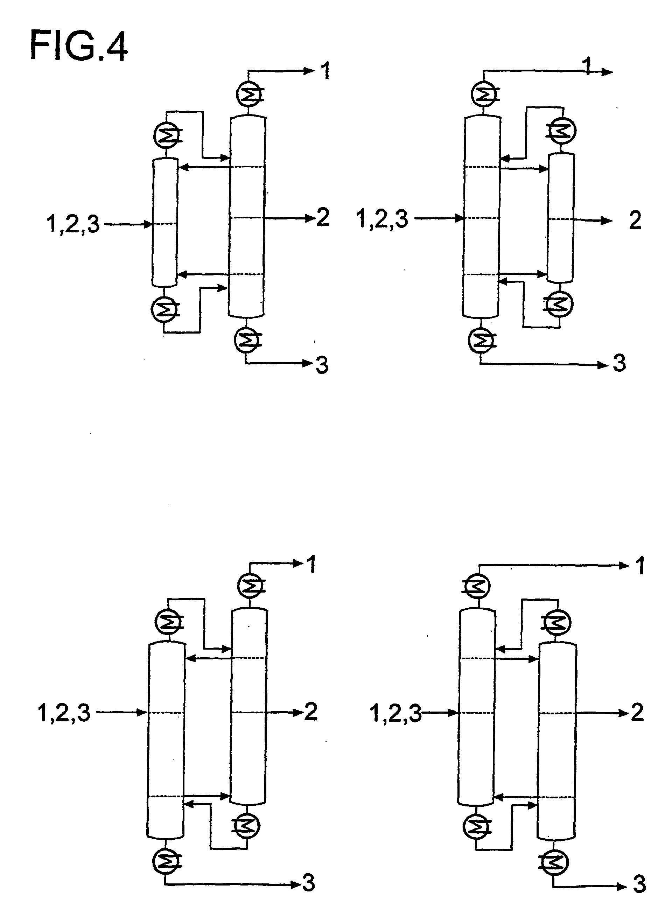

In Fig. 1 ein vereinfachtes Schema einer Trennwandkolonne sowie in Fig. 2, in Fig. 3 und in Fig. 4 vereinfachte Schemata von Systemen thermisch gekoppelter Destillationskolonnen.

Fig. 1 bis 4 zeigen die Auftrennung eines Vielstoffgemischs. Bei der Anwendung auf das erfindungsgemäße Verfahren wäre 1 Leichtsieder, 2 der höherschmelzende Stoff (als Mittelsieder) und 3 Schwersieder. Der Mittelsieder, also der höherschmelzende Stoff 2, wird als Reinstoff erhalten.

Im folgenden soll die Erfindung anhand von Ausführungsbeispielen näher erläutert werden:

### Beispiel 1

Die Destillation wurde mittels einer Laborkolonne mit einem Innendurchmesser von 50 mm durchgeführt. Der mittlere Teil der Kolonne war mit einer Trennwand in zwei symmetrische Teile getrennt. Der ungeteilte Abschnitt der Kolonne, unterhalb der Trennwand, wies eine Packungshöhe von 30 cm auf und war mit Gewebepackung (1200 m²/m³ Oberfläche) gefüllt. Der mittlere, geteilte Bereich der Kolonne (90 cm Höhe) war auf beiden Seiten der Trennwand mit Drahtringen (Durchmesser 3 mm) gefüllt. Über dem mittleren Teil der Kolonne war ein Schwenktrichter angebracht, der den Flüssigkeitsstrom im Aufteilungsverhältnis - Zulaufseite zu Abzugsseite von 3:7 (v/v) aufteilte. Durch den Schwenktrichter, der sich im Prinzip oben auf der Trennwand befindet, können die Anströmoberflächen der durch die Trennwand geteilten Bereiche variiert werden - der Schwenktrichter hat somit die Funktion einer "Klappe" die den Flüssigkeitsstrom in die beiden Bereiche verteilt. Der ungeteilte Abschnitt der Kolonne oberhalb der Trennwand (Höhe 60 cm) war mit Gewebepackung (1200 m²/m³) bestückt.

Die Destillation wurde bei einem Kopfdruck von 200 mbar durchgeführt. Die Sumpftemperatur wurde durch die Sumpfabzugsmenge auf 185 °C geregelt. Die Temperatur der Sumpfheizung betrug 205 °C. Die Seitenabzugsmenge wurde durch Regelung des Sumpfstandes eingestellt. Das Rücklaufverhältnis wurde durch Regelung der Temperatur in der Mitte des oberen Kolonnenbereichs eingestellt. Als Zulaufmenge wurden 150 g/h eingestellt. Die Regelungstemperatur im oberen Teil der Kolonne betrug 120 °C. Insgesamt wurden 3567 g eines Gemisches, enthaltend 60 Gew.-% Isobutanol, 13 Gew.-% o-Xylol, 13 Gew.-% 2,5-Dimethylhexan-2,5-diol und 8 Gew.-% Wasser, zugefahren. Der höherschmelzende, mittelsiedende Stoff 2 war dabei 2,5-Dimethylhexan-2,5-diol. Darüber hinaus waren im Zulaufgemisch verschiedene Spurenkomponenten, hauptsächlich Kondensationsprodukte des Acetons enthalten. Über Kopf wurden 3117 g eines Leichtsieders 1, ein Gemischs mit einem Restgehalt von 0,04 Gew.% 2,5-Dimethylhexan-2,5-diol, gewonnen. Im Seitenabzug wurden 476 g 2,5-Dimethylhexan-2,5-diol der Reinheit 99,72 Gew.-% gewonnen. Im Sumpf wurden nur sehr wenig Schwersieder 3 erhalten. Der höherschmelzende Stoff 2 2,5-Dimethylhexan-2,5-diol wurde an keiner Stelle der eingesetzten Kolonne als Feststoff abgeschieden.

### Beispiel 2

Der Versuch wurde analog Beispiel 1 durchgeführt.

Als Zulaufmenge wurden 150 g/h eingestellt. Die Sumpftemperatur betrug 202 °C. Das Zulaufgemisch enthielt: 30 Gew.-% Isobutanol, 23,8 Gew.-% 2,5-Dimethylhexan-2,5-diol, 34 Gew.-% o-Xylol, 2 Gew.-% Methylbutanol, 8 Gew.-% Wasser. Der höherschmelzende, mittelsiedende Stoff 2 war dabei 2,5-Dimethylhexan-2,5-diol. Darüber hinaus waren im Zulaufgemisch verschiedene Spurenkomponenten, hauptsächlich Kondensationsprodukte des Acetons enthalten. Über Kopf wurden 2636 g Leichtsieder 1 (Gehalt an 2,5-Dimethylhexam-2,5-diol etwa 2,2 Gew.%) abgezogen. Im Seitenabzug wurden 764 g 2,5-Dimethylhexan-2,5-diol mit einer Reinheit von 99,9 Gew.-% gewonnen. Im Sumpf wurden während des Versuchszeitraums 11 g Schwersieder 3 mit einem Gehalt von 17,1 Gew.-% abgezogen. Der höherschmelzende Stoff 2 2,5-Dimethylhexan-2,5-diol wurde an keiner Stelle der eingesetzten Kolonne als Feststoff.

## Patentansprüche

1. Verfahren zur kontinuierlich betriebenen destillativen Abtrennung eines höherschmelzenden Stoffes (2) aus einem den höherschmelzenden Stoff (2) und *einen* niedrig schmelzenden Leichtsieder (1) enthaltenden Ausgangsgemisch, wobei der *niedrig schmelzende* Leichtsieder (1) aus *einer* oder mehreren Komponenten mit einem niedrigeren Siedepunkt als der höherschmelzende Stoff (2) besteht, in einer Destillationsapparatur, die entweder als Trennwandkolonne oder als System thermisch gekoppelter Destillationskolonnen ausgebildet ist, **dadurch gekennzeichnet, daß** die Destillationsapparatur mit einem im Kopf, oberhalb der Entnahmestelle des höherschmelzenden Stoffes (2) befindlichen Kondensator ausgestattet ist, dessen Teilbereich der Oberfläche, der mit dem Innenraum der Destillationsapparatur Kontakt hat, eine niedrigere Temperatur aufweist als der Schmelzpunkt des höherschmelzenden Stoffes (2) und der Leichtsieder (1) im Kopf so stark *konzentriert ist*, daß keine Abscheidung des höherschmelzenden Stoffes (2) am Kondensator erfolgt.

2. Verfahren nach Anspruch 1, **dadurch gekennzeichnet, daß** das Ausgangsgemisch neben dem höherschmelzenden Stoff (2) und dem Leichtsieder (1) zusätzlich noch einen aus einer oder mehreren Komponenten bestehenden Schwersieder (3) enthält, wobei alle Komponenten des Schwersieders (3) einen höheren Siedepunkt als der höherschmelzende Stoff (2) aufweisen.

3. Verfahren nach Anspruch 1 oder 2, **dadurch gekennzeichnet, daß** die Destillationsapparatur nur einen Kondensator enthält.

4. Verfahren nach einem der Ansprüche 1 bis 3, **dadurch gekennzeichnet, daß** die Anreicherung des Leichtsieders (1) im Kopf durch
a) eine ausreichend groß bemessene Trennstufenzahl im oberen Bereich der Destillationsapparatur und/oder
b) eine hinreichend starke Beheizung der Destillationsapparatur und/oder
c) eine kontrollierte Entnahme des Leichtsieders (1) am Kopf
erreicht wird.

5. Verfahren nach einem der Ansprüche 1 bis 4, **dadurch gekennzeichnet, daß** die Anreicherung des Leichtsieders (1) im Kopf durch eine Temperaturregelung im oberen Bereich der Destillationsapparatur gesteuert wird.

6. Verfahren nach einem der Ansprüche 1 bis 5, **dadurch gekennzeichnet, daß** nur eine Teilmenge des Leichtsieders (1) dem Ausgangsgemisch als Fremdstoff vorliegt.

7. Verfahren nach einem der Ansprüche 1 bis 5, **dadurch gekennzeichnet, daß** das Ausgangsgemisch keine Fremdstoffe als Leichtsieder (1) enthält.

8. Verfahren nach einem der Ansprüche 1 bis 7, **dadurch gekennzeichnet, daß** die Destillationsapparatur entweder als Packungskolonne mit *Füllkörpern* oder geordneten Packungen oder als Bodenkolonne ausgebildet ist.

9. Verfahren nach einem der Ansprüche 1 bis 8, **dadurch gekennzeichnet, daß** als höherschmelzender Stoff (2) Isomere des Dimethylhexandiols eingesetzt werden.

10. Verfahren nach einem der Ansprüche 1 bis 9, **dadurch gekennzeichnet, daß** das Ausgangsgemisch als Leichtsieder (1) niedrig siedende Alkohole und/oder niedrig siedende aromatische Kohlenwasserstoffe und/oder niedrig siedende Ether enthält.

## Claims

1. A process for the continuous separation of a relatively high-melting material (2) from a starting mixture comprising the relatively high-melting material (2) and a low-melting low boiler (1), where the low-melting low boiler (1) consists of one or more components having a boiling point lower than that of the relatively high-melting material (2), by distillation in a distillation apparatus configured either as a dividing wall column or as a system of thermally coupled distillation columns, wherein the distillation apparatus is fitted with a condenser located at the top above the offtake point for the relatively high-melting material (2), where that part of the surface of this condenser which is in contact with the interior of the distillation apparatus has a temperature lower than the melting point of the relatively high-melting material (2) and the concentration of the low boiler (1) at the top becomes so high that no deposition of the relatively high-melting material (2) occurs in the condenser.

2. A process as claimed in claim 1, wherein the starting mixture comprises not only the relatively high-melting material (2) and the low boiler (1) but also a high boiler (3) consisting of one or more components, where all components of the high boiler (3) have a boiling point higher than that of the relatively high-melting material (2).

3. A process as claimed in claim 1 or 2, wherein the distillation apparatus has only one condenser.

4. A process as claimed in any of claims 1 to 3, wherein the increase in the concentration of the low boiler (1) at the top is achieved by
a) a sufficiently high number of theoretical plates in the upper region of the distillation apparatus and/or
b) sufficient heating of the distillation apparatus and/or
c) controlling the amount of low boiler (1) taken off at the top.

5. A process as claimed in any of claims 1 to 4, wherein the increase in the concentration of the low boiler (1) at the top is controlled by means of a temperature control in the upper region of the distillation apparatus.

6. A process as claimed in any of claims 1 to 5, wherein only part of the low boiler (1) is present as foreign substance in the starting mixture.

7. A process as claimed in any of claims 1 to 5, wherein the starting mixture contains no foreign substances as low boiler (1).

8. A process as claimed in any of claims 1 to 7, wherein the distillation apparatus is configured either as a packed column containing random packing elements or ordered packing or as a tray column.

9. A process as claimed in any of claims 1 to 8, wherein the relatively high-melting material (2) is composed of isomers of dimethylhexanediol.

10. A process as claimed in any of claims 1 to 9, wherein the starting material comprises, as low boiler (1), low-boiling alcohols and/or low-boiling aromatic hydrocarbons and/or low-boiling ethers.

## Revendications

1. Procédé pour séparer en continu, par distillation, une substance à haut point de fusion (2) à partir d'un mélange initial contenant la substance à haut point de fusion (2) et un constituant à bas point de fusion (1), ce dernier consistant en un ou plusieurs composants à point d'ébullition plus bas que celui de la substance à haut point de fusion (2) dans un appareil à distiller consistant en une colonne à paroi de séparation ou en un système de colonnes à distiller à couplage thermique, **caractérisé en ce que** l'appareil est équipé d'un condenseur qui se trouve en tête, au-dessus du point de prélèvement de la substance à haut point de fusion (2) et dont la région de la surface qui est en contact avec l'espace intérieur de l'appareil à distiller est à une température inférieure au point de fusion de la substance à haut point de fusion (2) et **en ce que** le constituant à bas point d'ébullition (1) est, en tête, si fortement concentré qu'il ne se produit pas de déposition de la substance à haut point de fusion (2) dans le condenseur.

2. Procédé selon la revendication 1, **caractérisé en ce que** le mélange initial contient encore, en plus de la substance à haut point de fusion (2) et du constituant à bas point d'ébullition (1), un constituant peu volatil (3) consistant en un ou plusieurs composants, tous les composants du constituant peu volatil (3) ayant un point d'ébullition supérieur à celui de la substance à haut point de fusion (2).

3. Procédé selon la revendication 1 ou 2, **caractérisé en ce que** l'appareil à distiller ne comprend qu'un seul condenseur.

4. Procédé selon l'une des revendications 1 à 3, **caractérisé en ce que** l'on parvient à enrichir en tête le constituant à bas point d'ébullition (1) par
a) un nombre suffisant d'étages de séparation dans la région supérieure de l'appareil à distiller et/ou
b) un chauffage suffisamment intense de l'appareil à distiller et/ou
c) un prélèvement contrôlé du constituant à bas point d'ébullition (1) en tête.

5. Procédé selon l'une des revendications 1 à 4, **caractérisé en ce que** l'on contrôle l'enrichissement du constituant à bas point d'ébullition (1) en tête par un réglage de température dans la région supérieure de l'appareil à distiller.

6. Procédé selon l'une des revendications 1 à 5, **caractérisé en ce qu'**une partie seulement du composant à bas point d'ébullition (1) est présente dans le mélange initial à l'état de constituant étranger.

7. Procédé selon l'une des revendications 1 à 5, **caractérisé en ce que** le mélange initial ne contient pas de constituants étrangers consistant en substances à bas point d'ébullition (1).

8. Procédé selon l'une des revendications 1 à 7, **caractérisé en ce que** l'appareil à distiller consiste en une colonne à corps de garnissage ou à garnissages ordonnés ou en une colonne à plateaux.

9. Procédé selon l'une des revendications 1 à 8, **caractérisé en ce que** le constituant à haut point de fusion (2) consiste en isomères du diméthylhexanediol.

10. Procédé selon l'une des revendications 1 à 9, **caractérisé en ce que** le mélange initial contient en tant que constituants à bas point d'ébullition (1) des alcools à bas point d'ébullition et/ou des hydrocarbures aromatiques à bas point d'ébullition et/ou des éthers à bas point d'ébullition.
